(19) Europäisches Patentamt
European Patent Office
Office européen des brevets

(11) EP 1 444 990 A1

(12) **EUROPEAN PATENT APPLICATION**

(43) Date of publication:
**11.08.2004 Bulletin 2004/33**

(51) Int Cl.[7]: **A61K 51/04**, A61K 49/00, A61K 51/12, C07D 451/02

(21) Application number: **03002809.6**

(22) Date of filing: **07.02.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(71) Applicant: **Amersham plc**
**Little Chalfont Buckinghamshire HP7 9NA (GB)**

(72) Inventors:
- **McGill, David**
**Amersham, Bucks HP7 9LL (GB)**
- **Henriksen, Ingrid**
**Postboks 4220 Nydalen, N-0401 Oslo (NO)**

(74) Representative: **Canning, Lewis R. et al**
**Amersham plc**
**Amersham Place**
**Little Chalfont, Buckinghamshire HP7 9NA (GB)**

Remarks:
Claims 11-28 are deemed to be abandoned due to non-payment of the claims fees (Rule 31 (2) EPC).

(54) **Improved Radiometal Complex Compositions**

(57) The present invention relates to stabilised technetium and rhenium metal complex compositions comprising a radioprotectant and a radiometal complex of a tropane-tetradentate chelating agent conjugate, wherein the radiometal complex is neutral. Radiopharmaceuticals comprising the stabilised metal complex compositions, and kits for the preparation of the radiopharmaceuticals are also described.

EP 1 444 990 A1

Printed by Jouve, 75001 PARIS (FR)

## Description

Field of the Invention.

The present invention relates to stabilised technetium and rhenium metal complex compositions comprising a radioprotectant and a radiometal complex of a tropane-tetradentate chelating agent conjugate. Radiopharmaceuticals comprising the stabilised metal complex compositions, and kits for the preparation of the radiopharmaceuticals are also described.

Background to the Invention.

Tropanes labelled with $^{123}I$, $^{18}F$ or $^{99m}Tc$ are known as diagnostic imaging radiopharmaceuticals for brain imaging [Morgan and Nowotnik, Drug News Perspect. 12(3), 137-145 (1999)]. Tropanes are known to target the dopamine transporter in the brain, and the dopamine transporter has been implicated in several diseases including Parkinson's Disease, Parkinsonian Syndrome and attention-deficit hyperactivity disorder.

Tropanes labelled with $^{99m}Tc$ are known. The development of $^{99m}Tc$-TRODAT-1 has been described by Kung [Nucl.Med.Biol., 28, p.505-508 (2001)]:

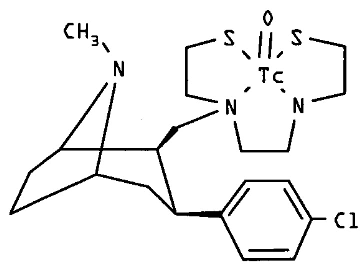

$^{99m}$Tc-TRODAT-1

TRODAT is also described in US 5980860 and equivalents.

Technepine has also been described by Meltzer *et al* [J.Med.Chem., 40, 1835-1844 (1997)]:

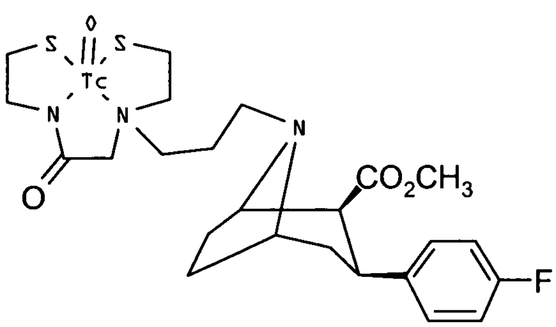

Technepine

Technepine is described in US 6171576 and equivalents.

A range of $^{99m}Tc$ complexes of $N_2S_2$ diaminedithiol chelator conjugates of tropanes (including TRODAT-1) have been reported to show good *in vitro* stability at 4 and 24 hours post preparation, with little change in radiochemical purity [Meegalla *et al*, J.Med.Chem., 40, p.9-17 (1997)]. Fan *et al* [Chin.J.Nucl.Med., 19(3) 146-148 (1999)] report that $^{99m}Tc$-TRODAT-1 is stable for 24 hours at room temperature.

An improved kit formulation for the preparation of $^{99m}Tc$-TRODAT-1 has been described [Choi *et al*, Nucl.Med.

Biol., 26, p. 461-466 (1999)]. Choi *et al* report that, as long as a minimum of 10 µg (microgrammes) of the tropane conjugate is present in the kit, the radiochemical purity consistently reaches greater than 90%. Heating is necessary to achieve a satisfactory radiochemical purity (RCP), and Choi *et al* use autoclave heating for 30 minutes.

The Present Invention.

The present invention provides improved radiometal complex compositions comprising a technetium or rhenium metal complex of a tropane-tetradentate chelating agent conjugate and a radioprotectant. The improved compositions exhibit more reproducible initial radiochemical purity (RCP) and improved stability post-reconstitution, so that an RCP of 85 to 90% is maintained at 4 hours post-reconstitution. The problem of unsatisfactory RCP for radiometal tropane conjugates under certain conditions of radioactivity levels, radioactive concentrations or reconstitution volumes was not recognised in the prior art. Such conditions are those that could arise under normal conditions of use of a commercial radionuclide generator, such as a $^{99m}$Tc generator. The present invention provides compositions comprising a radioprotectant which solve this previously unrecognised problem.

Detailed Description of the Invention.

In a first embodiment, the present invention provides a stabilised composition which comprises:

(i) a metal complex of a radioactive isotope of technetium or rhenium chelated to a conjugate, wherein said conjugate comprises a tetradentate chelating agent conjugated to a tropane, and said tetradentate chelating agent forms a neutral metal complex with said radioactive isotope of technetium or rhenium;
(ii) at least one radioprotectant.

By the term "metal complex" is meant a coordination complex of the technetium or rhenium metal ion with a ligand, here the tetradentate chelating agent. The chelated metal complex is "resistant to transchelation", ie. does not readily undergo ligand exchange with other potentially competing ligands for the radiometal coordination sites. Potentially competing ligands include the tropane moiety itself, the radioprotectant or other excipients in the preparation *in vitro* (eg. antimicrobial preservatives), or endogenous compounds *in vivo* (eg. glutathione, transferrin or plasma proteins).
Suitable radioactive isotopes of technetium or rhenium include: $^{94m}$Tc, $^{99m}$Tc, $^{186}$Re and $^{188}$Re. A preferred such radioisotope is $^{99m}$Tc.
The term "tetradentate" has its' conventional meaning, ie. the chelating agent has 4 metal donor atoms which coordinate to the metal giving chelate rings on formation of the metal complex.
The technetium and rhenium metal complexes of the present invention are "neutral", ie. any positive charge on the central metal core is balanced by the sum of the negative charge on the four metal donor atoms of the tetradentate chelating agent, to give an overall electrically neutral metal complex. Examples of likely technetium cores are $O=Tc^{+}=O$ and $Tc^{3+}=O$, which both represent technetium in the Tc(V) oxidation state.
The neutral radioactive technetium or rhenium complexes of the present invention are suitably of Formula I:

$$[\{tropane\}-(A)_n]_m-[metal\ complex] \quad (I)$$

where:

$(A)_n$ is a linker group,
n is an integer of value 0 to 10,
and m is 1, 2 or 3.

The "linker group" $(A)_n$ is as defined below for Formula Ia. The metal complexes of Formula I are derived from tropane "conjugates". The tropane tetradentate chelating agent "conjugates" of the present invention are as defined in Formula Ia:

$$[\{tropane\}-(A)_n]_m-[tetradentate\ chelating\ agent] \quad (Ia)$$

where:

$-(A)_n-$ is a linker group wherein each A is independently $-CR_2-$ , -CR=CR-, -C≡C-, $-CR_2CO_2-$, $-CO_2CR_2-$, -NRCO-, -CONR-, -NR(C=O)NR-, - NR(C=S)NR-, $-SO_2NR-$, $-NRSO_2-$, $-CR_2OCR_2-$, $-CR_2SCR_2-$, $-CR_2NRCR_2-$, a $C_{4-8}$ cycloheteroalkylene group, a $C_{4-8}$ cycloalkylene group, a $C_{5-12}$ arylene group, or a $C_{3-12}$ heteroarylene group;
R is independently chosen from H, $C_{1-4}$ alkyl, $C_{2-4}$ alkenyl, $C_{2-4}$ alkynyl, $C_{1-4}$ alkoxyalkyl or $C_{1-4}$ hydroxyalkyl;
n is an integer of value 0 to 10; and
m is 1, 2 or 3.

In Formulae I and Ia, m is preferably 1 or 2, and is most preferably 1; and $(A)_n$ is preferably $(CR_2)_n$, where n is chosen to be 1 to 3.

Examples of suitable tetradentate chelating agents for technetium and rhenium which form neutral metal complexes include, but are not limited to:

(i) $N_2S_2$ ligands having a diaminedithiol donor set such as BAT, an amideaminedithiol donor set such as MAMA, a phenylenediaminethioetherthiol donor set such as PhAT, or a dithiosemicarbazone donor set;

(ii) diaminedioximes;

(iii) $N_3S$ ligands having a diamidepyridinethiol donor set such as Pica;

(iv) open chain or macrocyclic ligands having an amidetriamine donor set, such as monoxocyclam.

(v) $N_2O_2$ ligands having a diaminediphenol donor set.

The above described ligands are particularly suitable for complexing technetium eg. $^{94m}$Tc or $^{99m}$Tc, and are described more fully by Jurisson *et al* [Chem.Rev., 99, 2205-2218 (1999)]. $N_2S_2$ dithiosemicarbazone chelators are described by Arano *et al* [Chem.Pharm.Bull., 39, p.104 - 107 (1991)]. $N_2S_2$ phenylenediaminethioetherthiol chelators are described by McBride *et al* [J.Med.Chem., 36, p.81-6 (1993)]. Macrocyclic amidetriamine ligands and their tropane conjugates are described by Turpin *et al* [J.Lab.Comp.Radiopharm., 45, 379-393 (2002)]. Diaminedioximes are described by Nanjappan *et al* [Tetrahedron, 50, 8617-8632 (1994)]. $N_3S$ ligands having a diamidepyridinethiol donor set are described by Bryson *et al* [Inorg.Chem., 29, 2948-2951 (1990)]. $N_2O_2$ ligands having a diaminediphenol donor set are described by Pillai *et al* [Appl.Rad.Isot., 41, 557-561 (1990)].

Preferred $^{99m}$Tc metal complexes of the present invention are those suitable for crossing the blood-brain barrier (BBB) as described by Volkert *et al* [Radiochim. Acta, 63, p.205-208 (1993)]. Especially preferred $^{99m}$Tc metal complexes of the present invention are $^{99m}$Tc-TRODAT and Technepine.

Preferred tetradentate chelating agents are those having an $N_2S_2$ diaminedithiol or amideaminedithiol donor set of Formula II:

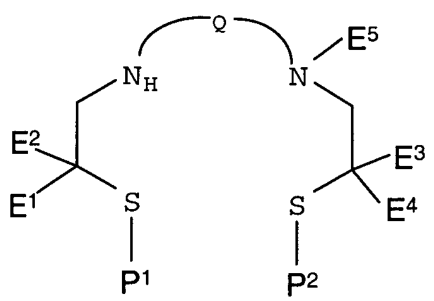

II

where:

$E^1$-$E^5$ are each independently an R' group;
each R' is H or $C_{1-10}$ alkyl, $C_{3-10}$ alkylaryl, $C_{2-10}$ alkoxyalkyl, $C_{1-10}$ hydroxyalkyl, $C_{1-10}$ fluoroalkyl, $C_{2-10}$ carboxyalkyl or $C_{1-10}$ aminoalkyl, or two or more R' groups together with the atoms to which they are attached form a carbocyclic, heterocyclic, saturated or unsaturated ring, and wherein one or more of the R' groups is conjugated to the tropane;
and Q is a bridging group of formula $-J(CR'_2)_f-$ ;

where f is 1 or 2, and J is $-CR'_2-$ or C=O;
$P^1$ and $P^2$ are independently H or a thiol protecting group.

By the term "protecting group" is meant a group which inhibits or suppresses undesirable chemical reactions (eg. oxidation of the free thiol to the corresponding disulphide), but which is designed to be sufficiently reactive that it may be cleaved from the thiol under mild enough conditions that do not modify the rest of the molecule during radiolabelling of the conjugate. Thiol protecting groups are well known to those skilled in the art and include but are not limited to: trityl, 4-methoxybenzyl, benzyl, tetrahydropyranyl, include but are not limited to: trityl, 4-methoxybenzyl, benzyl, tetrahydropyranyl, methyltetrahydrofuranyl (MTHF), acetamidomethyl and ethoxyethyl. The use of further thiol protecting groups are described in 'Protective Groups in Organic Synthesis', Theorodora W. Greene and Peter G. M. Wuts, (John Wiley & Sons, 1991). In Formula II, preferably both $P^1$ and $P^2$ are H.
Preferred Q groups are as follows: $-CH_2CH_2-$ , $-CH_2CH_2CH_2-$ or $-(C=O)CH_2-$, most preferably $-CH_2CH_2-$ , $-CH_2CH_2CH_2-$, with $-CH_2CH_2-$ being especially preferred.
$E^1$ to $E^5$ are preferably chosen from: H, $C_{1-3}$ alkyl, $C_{1-3}$ alkoxyalkyl, $C_{1-3}$ hydroxyalkyl or $C_{1-3}$ fluoroalkyl. Most preferably, each $E^1$ to $E^4$ group is H, and $E^5$ is $C_{1-3}$ alkyl.
A most particularly preferred chelator of Formula II is the $N_2S_2$ diaminedithiol of TRODAT-1.
The tetradentate chelating agents of Formula II are preferably attached to the tropane *via* either the bridging group Q or the $E^5$ group. Most preferably, the tropane is attached *via* the $E^5$ group.
The term "tropane" has its' conventional meaning, ie. a bicyclic amine of formula (with numbering of the ring positions shown):

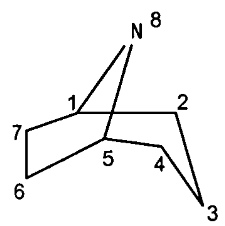

where the amine nitrogen at the 8-position may be secondary or tertiary. The tetradentate chelating agent is preferably attached at either the 2- or the 8-position of the tropane, most preferably at the 2-position.
Preferably, the tropane of the present invention is a phenyl tropane of Formula III:

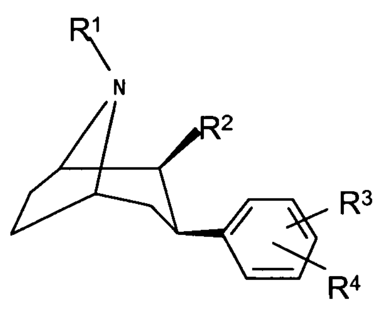

(III)

where:

$R^1$ is H, $C_{1-4}$ alkyl, $C_{1-4}$ alkenyl or $C_{1-4}$ fluoroalkyl;
$R^2$ is $CO_2R^5$, $CON(R^5)_2$, $COR^5$ or $C_{1-4}$ alkyl, where each $R^5$ is independently H or $C_{1-3}$ alkyl;
$R^3$ and $R^4$ are independently H, Cl, Br, F, I, $CH_3$, $C_2H_5$, $CF_3$, $NO_2$, $OCH_3$ or $NH_2$.

$R^1$ is preferably $C_{1-3}$ alkyl or $C_{1-3}$ fluoroalkyl. $R^2$ is preferably $CO_2CH_3$ or $C_{1-2}$ alkyl. $R^3$ is preferably 4-chloro,

4-fluoro or 4-methyl, and $R^4$ is preferably H or $CH_3$. $R^1$ is most preferably $CH_3$.

By the term "radioprotectant" is meant a compound which inhibits degradation reactions induced by radioactive emissions, such as redox processes, by trapping highly-reactive free radicals, such as oxygen-containing free radicals arising from the radiolysis of water. The radioprotectants of the present invention are suitably chosen from: ascorbic acid, *para*-aminobenzoic acid (ie. 4-aminobenzoic acid), gentisic acid (ie. 2,5-dihydroxybenzoic acid) and salts thereof with a biocompatible cation. Preferred radioprotectants are ascorbic acid and *para*-aminobenzoic acid, or salts thereof with a biocompatible cation. Especially preferred radioprotectants are ascorbic acid and salts thereof with a biocompatible cation. A preferred such salt is sodium ascorbate. The radioprotectants of the present invention are commercially available to a pharmaceutical grade specification.

By the term "biocompatible cation" is meant a positively charged counterion which forms a salt with an ionised, negatively charged group, where said positively charged counterion is also non-toxic and hence suitable for administration to the mammalian body, especially the human body. Examples of suitable biocompatible cations include: the alkali metals sodium or potassium; the alkaline earth metals calcium and magnesium; and the ammonium ion. Preferred biocompatible cations are sodium and potassium, most preferably sodium.

It is envisaged that the role of the linker group $-(A)_n-$ of Formula I is to distance the relatively bulky metal complex, from the tropane, so that binding of the tropane to biological target sites (eg. the dopamine transporter in the mammalian brain) is not impaired. This can be achieved by a combination of flexibility (eg. simple alkyl chains), so that the bulky group has the freedom to position itself away from the active site and/or rigidity such as a cycloalkyl or aryl spacer which orientates the metal complex away from the active site.

The nature of the linker group can also be used to modify the biodistribution of the resulting metal complex of the conjugate. Thus, eg. the introduction of ether groups in the linker will help to minimise plasma protein binding. Preferred linker groups $-(A)_n-$ have a backbone chain of linked atoms which make up the $-(A)_n-$ moiety of 2 to 10 atoms, most preferably 2 to 5 atoms, with 2 or 3 atoms being especially preferred. A minimum linker group backbone chain of 2 atoms confers the advantage that the chelator is well-separated from the tropane, so that any interaction is minimised.

Non-peptide linker groups such as alkylene groups or arylene groups have the advantage that there are no significant hydrogen bonding interactions with the conjugated tropane, so that the linker does not wrap round onto the tropane. Preferred alkylene spacer groups are $-(CH_2)_q-$ where q is 2 to 5. Preferred arylene spacers are of formula:

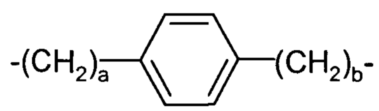

where: a and b are independently 0, 1 or 2.

It is strongly preferred that the tropane is bound to the metal complex in such a way that the linkage does not undergo facile metabolism in blood, since that would result in the metal complex being cleaved off before the labelled tropane inhibitor reached the desired *in vivo* target site. The tropane is therefore preferably covalently bound to the metal complexes of the present invention *via* linkages which are not readily metabolised.

The concentration of radioprotectant for use in the present invention is suitably 0.3 to 5.0 millimolar, preferably 0.5 to 4.0 millimolar, most preferably 1.0 to 3.5 millimolar. For ascorbic acid, this corresponds to a suitable concentration of 50 to 900 $\mu g/cm^3$, preferably 70 to 800 $\mu g/cm^3$, most preferably 90 to 700 $\mu g/cm^3$. For the $^{99m}Tc$ radiopharmaceutical $^{99m}Tc$-TRODAT, the preferred concentration of an ascorbic acid or ascorbate radioprotectant is in the range 0.5 to 3.8 millimolar.

The stabilised composition of the present invention may be prepared by reacting a solution of the radiometal in the appropriate oxidation state with the conjugate at the appropriate pH in the presence of the radioprotectant, in solution in a suitable solvent. The radioprotectant may be supplied either together with the conjugate or the solution of the radiometal. Preferably, the radioprotectant is pre-mixed with the conjugate, and that mixture is subsequently reacted with the radiometal. The conjugate solution may preferably contain a ligand which complexes weakly, but rapidly to the radiometal (such as gluconate or citrate) i.e. the radiometal complex is prepared by ligand exchange or transchelation. Such conditions are useful to suppress undesirable side reactions such as hydrolysis of the metal ion. When the radiometal is rhenium, the usual radioactive starting material is perrhenate, ie. $ReO_4^-$. When the radiometal is $^{99m}Tc$, the usual radioactive starting material is sodium pertechnetate from a $^{99}Mo$ generator. In both perrhenate and pertechnetate the metal (M) is present in the M(VII) oxidation state, which is relatively unreactive. The preparation of technetium or rhenium complexes of lower oxidation state M(I) to M(V) therefore usually requires the addition of a suitable pharmaceutically acceptable reducing agent such as sodium dithionite, sodium bisulphite, ascorbic acid, formamidine sulphinic acid, stannous ion, Fe(II) or Cu(I), to facilitate complexation. The pharmaceutically acceptable

reducing agent is preferably a stannous salt, most preferably stannous chloride, stannous fluoride or stannous tartrate.

Alternatively, the stabilised composition of the present invention may be prepared in a stepwise manner by first forming the radiometal complex in a suitable solvent, and subsequently adding the radioprotectant. In such an approach, the radioprotectant should be added as soon as possible after formation of the radiometal complex, so that the stabilising effect of the radioprotectant is brought into effect to minimise radiolysis and possible degradation. Methods of preparation wherein the radioprotectant is present prior to formation of the radiometal complex are preferred.

When the radiometal complexes of the present invention are to be used in radiopharmaceutical compositions, a preferred method of preparation is the use of a sterile, non-radioactive kit as described in the third and fourth embodiments below. The kit provides a convenient supply of the necessary reactants at the right concentration, which needs only be reconstituted with perrhenate or pertechnetate in saline or another suitable solvent.

In a second embodiment, the present invention provides a conjugate useful in the preparation of the above stabilised composition, said conjugate being of Formula Ia as defined above. Preferably, the tropane of the conjugate is a phenyl tropane of Formula III (above). Preferred and most preferred phenyl tropanes for the conjugate are as described above for the first embodiment. Most preferably, the conjugate is of Formula IV:

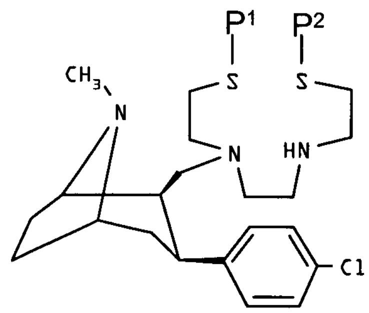

(IV)

where $P^1$ and $P^2$ are independently H or a thiol protecting group.

The term "protecting group" is as defined for Formula II above. Preferred conjugates of Formula IV are where $P^1$ and $P^2$ are both H.

The conjugates of the present invention may be prepared *via* the bifunctional chelate approach. Thus, it is well known to prepare chelating agents which have attached thereto a functional group ("bifunctional chelates"). Functional groups that have been attached include: amine, thiocyanate, maleimide and active esters such as N-hydroxysuccinimide or pentafluorophenol. Such bifunctional chelates can be reacted with suitable functional groups on the tropane to form the desired conjugate. Such suitable functional groups on the tropane include:

carboxyls (for amide bond formation with an amine-functionalised bifunctional chelator);
amines (for amide bond formation with an carboxyl- or active ester-functionalised bifunctional chelator);
halogens, mesylates and tosylates (for N-alkylation of an amine-functionalised bifunctional chelator) and
thiols (for reaction with a maleimide-functionalised bifunctional chelator). Further details of the bifunctional chelate approach are described by Arano [Adv.Drug.Deliv.Rev., 37, 103-120 (1999)]. Further details specific to the conjugation of tropanes with the tetradentate chelating agents indicated are described in: the methods of Meegalla *et al* [J.Med.Chem., 40, 9-17 (1997)] for $N_2S_2$ diaminedithiol chelators; Meltzer *et al* for $N_2S_2$ amideaminedithiol (MAMA) chelators [*ibid*, 40, 1835-1844 (1997)] and Turpin *et al* [J.Lab.Comp.Radiopharm., 45, 379-393 (2002)] for monoxocyclam chelators.

In a third embodiment, the present invention provides a radiopharmaceutical which comprises the stabilised composition of the first embodiment together with a biocompatible carrier, in a form suitable for mammalian administration. The "biocompatible carrier" is a fluid, especially a liquid, in which the imaging agent can be suspended or dissolved, such that the composition is physiologically tolerable, ie. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is either isotonic or not hypotonic); an aqueous solution of one or more tonicity-adjusting substances (eg. salts of plasma cations with biocompatible counterions), sugars (e.g. glucose or sucrose), sugar alcohols (eg. sorbitol or mannitol), glycols (eg. glycerol), or other non-ionic polyol materials (eg. polyethyleneglycols, propylene

glycols and the like).

The radiopharmaceuticals of the present invention may optionally further comprise an antimicrobial preservative. By the term "antimicrobial preservative" is meant an agent which inhibits the growth of potentially harmful micro-organisms such as bacteria, yeasts or moulds. The antimicrobial preservative may also exhibit some bactericidal properties, depending on the dose. The main role of the antimicrobial preservative(s) of the present invention is to inhibit the growth of any such micro-organism in the radiopharmaceutical composition post-reconstitution, ie. in the radioactive diagnostic product itself. Suitable antimicrobial preservative(s) include: the parabens, ie. methyl, ethyl, propyl or butyl paraben or mixtures thereof; benzyl alcohol; phenol; cresol; cetrimide and thiomersal. Preferred antimicrobial preservative(s) are the parabens.

Such radiopharmaceuticals are suitably supplied in either a container which is provided with a seal which is suitable for single or multiple puncturing with a hypodermic needle (e.g. a crimped-on septum seal closure) whilst maintaining sterile integrity. Such containers may contain single or multiple patient doses. Preferred multiple dose containers comprise a single bulk vial (e.g. of 10 to 30 $cm^3$ volume) which contains multiple patient doses, whereby single patient doses can thus be withdrawn into clinical grade syringes at various time intervals during the viable lifetime of the preparation to suit the clinical situation. Pre-filled syringes are designed to contain a single human dose, and are therefore preferably a disposable or other syringe suitable for clinical use. The pre-filled syringe may optionally be provided with a syringe shield to protect the operator from radioactive dose. Suitable such radiopharmaceutical syringe shields are known in the art and preferably comprise either lead or tungsten.

When the radioactive isotope is $^{99m}Tc$, a radioactivity content suitable for a diagnostic imaging radiopharmaceutical is in the range 180 to 1500 MBq of $^{99m}Tc$, depending on the site to be imaged *in vivo,* the uptake and the target to background ratio. $^{99m}Tc$ is suitable for SPECT imaging and $^{94m}Tc$ for PET imaging.

The radiopharmaceuticals of the present invention comprise the improved radiometal compositions of the first embodiment. This has the advantage that radioactive impurities are suppressed. Such impurities may either contribute to unnecessary radiation does for the patient, or may in some cases have an adverse effect on imaging by reducing the signal to background ratio.

The radiopharmaceuticals of the present invention may be prepared from kits, as is described in the fourth embodiment below. Alternatively, the radiometal complexes of the present invention in a biocompatible carrier may be prepared under aseptic manufacture conditions to give the desired sterile product. The radiopharmaceuticals may also be prepared under non-sterile conditions followed by terminal sterilisation, using eg. gamma irradiation, autoclaving, dry heat or chemical treatment (eg. with ethylene oxide). Preferably, the radiopharmaceuticals of the present invention are prepared from kits.

In a fourth embodiment, the present invention provides a kit for the preparation of the radiopharmaceuticals of the present invention, which comprises:

(i) the conjugate of Formula (Ia) or a salt thereof with a biocompatible counterion;
(ii) a radioprotectant (as defined above);
(iii) a biocompatible reductant.

Such kits are designed to give sterile radiopharmaceutical products suitable for human administration, e.g. *via* direct injection into the bloodstream. For $^{99m}Tc$, the kit is preferably lyophilised and is designed to be reconstituted with sterile $^{99m}Tc$-pertechnetate ($TcO_4^-$) from a $^{99m}Tc$ radioisotope generator to give a solution suitable for human or mammalian administration without further manipulation. Suitable kits comprise a container (eg. a septum-sealed vial) containing the conjugate (Ia) in either free base or acid salt form, together with a "biocompatible reductant" such as sodium dithionite, sodium bisulphite, ascorbic acid, formamidine sulphinic acid, stannous ion, Fe(II) or Cu(I). The biocompatible reductant is preferably a stannous salt such as stannous chloride or stannous tartrate.

The "radioprotectant" of the kit is as defined above. Preferred radioprotectants correspond to those described for the stabilised composition of the first embodiment.

The conjugate of Formula (Ia) comprises the amine of the tropane 8-position, plus possibly further amine donor atoms of the tetradentate chelating agent. Hence, the conjugate may optionally be used in the kit as "a salt thereof with a biocompatible counterion", ie. an acid salt of the conjugate. Suitable such salts include but are not limited to: hydrochlorides, trifluoroacetates, sulphonates, tartrates, oxalates and sulphosalicyclates. When the conjugate is of Formula IV, preferred salts are the trifluoroacetate or hydrochloride salts, especially the trifluoroacetate salt.

The non-radioactive kits may optionally further comprise additional components such as a transchelator, antimicrobial preservative, pH-adjusting agent or filler. The "transchelator" is a compound which reacts rapidly to form a weak complex with technetium, then is displaced by the ligand. This minimises the risk of formation of reduced hydrolysed technetium (RHT) due to rapid reduction of pertechnetate competing with technetium complexation. Suitable such transchelators are salts of a weak organic acid, ie. an organic acid having a pKa in the range 3 to 7, with a biocompatible cation. Suitable such weak organic acids are acetic acid, citric acid, tartaric acid, gluconic acid, glucoheptonic acid, benzoic acid, phenols or phosphonic acids. Hence, suitable salts are acetates, citrates, tartrates, gluconates, glucoheptonates, benzoates, phenolates or phosphonates. Preferred such salts are tartrates, gluconates, glucoheptonates, benzoates, or phosphonates, most preferably phosphonates, most especially diphosphonates. A preferred such transchelator is a gluconate or glucoheptonate salt with a biocompatible cation.

The "antimicrobial preservative" is as defined for the radiopharmaceutical embodiment (above). For the kit, the inclusion of an antimicrobial preservative means that, once reconstituted, the growth of potentially harmful microorganisms in the preparation is inhibited.

The term "pH-adjusting agent" means a compound or mixture of compounds useful to ensure that the pH of the reconstituted kit is within acceptable limits (approximately pH 4.0 to 10.5) for human or mammalian administration. Suitable such pH-adjusting agents include pharmaceutically acceptable buffers, such as tricine, phosphate or TRIS [ie. *tris*(hydroxymethyl)aminomethane], and pharmaceutically acceptable bases such as sodium carbonate, sodium bicarbonate or mixtures thereof. When the conjugate is employed in acid salt form, the pH adjusting agent may optionally be provided in a separate vial or container, so that the user of the kit can adjust the pH as part of a multistep procedure.

By the term "filler" is meant a pharmaceutically acceptable bulking agent which may facilitate material handling during production and lyophilisation. Suitable fillers include inorganic salts such as sodium chloride, and water soluble sugars or sugar alcohols such as sucrose, maltose, mannitol or trehalose. A preferred filler is mannitol.

In a fifth embodiment, the present invention provides a method of preparation of the radiopharmaceutical of the second embodiment, which comprises forming the metal complex of the first embodiment in a biocompatible carrier in a form suitable for mammalian administration, and subsequently adding an effective amount of one or more radioprotectants (as defined above).

In a sixth embodiment, the present invention provides the use of the radiopharmaceutical of the second embodiment in a method of diagnostic imaging of the mammalian brain.

In a further embodiment, the present invention provides a method of diagnostic imaging of the mammalian brain which comprises imaging a mammal which had previously been administered with the radiopharmaceutical of the second embodiment.

The invention is illustrated by the following non-limiting Examples. Example 1 describes the materials and methods used in the comparative studies described in later Examples. A formulation of the present invention (Formulation P) is compared with a prior art formulation (Formulation Q). Example 2 describes the radiolabelling protocol and quality control methodology employed. Example 3 shows that, under elution conditions 1, both formulations gave acceptable initial RCPs (92 and 88% for P and Q respectively) under these 'best case' generator elution conditions. Formulation P gave an RCP of 90% at 3.5 hours post-preparation, whereas for Formulation Q, the RCP fell off sharply to 77% over the same period of time. These results show that Formulation P exhibits both an improved initial RCP and an improved post-preparation stability.

Under generator elution conditions 2, Formulation P had an RCP of 92% immediately after preparation and 90% at 4 hours. The initial RCP for Formulation Q was 88%, but again it fell off significantly to 77% at 3.5 hours. These results demonstrate that even with generator eluate at the end of its' usable shelf-life, Formulation P displays an improved initial RCP, and greater post-preparation stability than prior art Formulation Q.

Under generator elution conditions 3, in spite of a long interval between generator elutions, both formulations give acceptable initial RCPs (91 and 88% for P and Q respectively). At 4 hours post-preparation the RCP of Formulation P is 88%, whereas the RCP of the Formulation is Q 73% after only 2 hours.

Under generator elution conditions 4, Formulation P exhibits an RCP of 90% after 1.5 hours and 89% at 3.5 hours post-preparation. When subjected to these testing eluate conditions, the RCP of prior art Formulation Q is only 76% at 1.5 hours, falling to 71 % at 3.5 hours.

Example 4 shows that p-ABA is also effective as a radioprotectant for $^{99m}$Tc-TRODAT preparations. Addition of p-ABA led to a significant improvement in RCP. Increasing the level of p-ABA from 200 to 500 µg increased further the stability at both initial and 4 hours.

Example 5 studies the effect of the volume of $^{99m}$Tc-pertechnetate used to reconstitute the kit vial ("reconstitution volume") on the RCP, at the same eluate radioactive concentration (0.75 GBq/ml). At the standard 2 ml reconstitution volume the Formulation P kits perform better. The Formulation P kits continue to radiolabel well even when reconstituted with 3 GBq in 4 ml, but the RCP drops markedly when kits are reconstituted with 4.5 GBq in 6 ml. These results show that the RCP of both formulations are effected by reconstitution volume. This effect may be attributable to an increased path length effect for radiolysis of the solvent. The inclusion of the radioprotectant ascorbate in Formulation P suppresses the volume effect compared to the prior art Formulation (Q), but does not eliminate it completely.

Example 6 studies the effect of use of an autoclave heating cycle (121 C, 30 minutes) as part of the radiolabelling procedure, since Choi *et al* [Nucl.Med.Biol., 26, p. 461-466 (1999)] employ that vial heating methodology. The present experiments were typically conducted using heating *via* a boiling water bath, since the use of an autoclave as part of the preparation procedure is not an attractive option for a commercial product. Hence, a comparative study was

carried out to determine if the different heating procedure might contribute to the RCP differences observed for Formulations P and Q. Example 6 indicates that the use of an autoclave heating cycle has a detrimental effect on the RCP of both formulations. Low RCPs were observed for both formulations at both analysis time points and high levels of hydrophilic impurities were seen in the radioactive HPLC chromatograms. Hence, the reported stability of the prior art Choi *et al* $^{99m}$Tc-TRODAT-1 preparations cannot be ascribed to the use of autoclaving.

Example 7 shows that the addition of the radioprotectant ascorbate to a $^{99m}$Tc-TRODAT preparation has a positive effect on the regional brain biodistribution properties, of $^{99m}$Tc-TRODAT. The apparent differences in blood retention and in selective retention in the striatum may be attributed to either the increased RCP achieved with the radioprotectant-containing formulation and/or the altered ratio of A/B diastereomers.

Experimental.

A series of comparative experiments has been carried out to generate radiolabelling data for the present formulation *vs* the optimised one for $^{99m}$Tc-TRODAT described in the prior art. The present formulation demonstrates significant advantages over the prior art published TRODAT formulation [Choi *et al*, Nucl.Med.Biol., 26, p. 461-466 (1999)].

**Example 1: Materials and methods.**

All studies were carried out using lyophilised kit formulations, $^{99m}$Tc-pertechnetate eluate from Amertec II™ generators. The kit vials were prepared under the same conditions but to different formulations - that of the present invention (Formulation P), and that of the prior art Choi *et al* formulation (Formulation Q). All vials were stored upright, in the dark at -20 °C until required for use. The formulations are given in Table 1:

Table 1:

| Present Kit Formulation (P) *vs* that of the Prior Art (Q). | | |
|---|---|---|
| **Kit component** | **Quantity of component per vial** | |
| | **Formulation P** | **Formulation Q (prior art)** |
| TRODAT-1 | 10 µg (as 18 µg of the trifluoroacetate salt) | 10 µg |
| $SnCl_2.2H_2O$ | 38 µg | 32 µg |
| Na-Gluconate | 10 mg | 10 mg |
| $Na_2EDTA.2H_2O$ | 840 µg | 840 µg |
| Na-Ascorbate | 500 µg | 0 |
| The most significant difference is that Formulation P contains a radioprotectant (sodium ascorbate), whereas Formulation Q does not. | | |

**Example 2: Radiolabelling procedure and purity determination.**

Unless otherwise stated, all test items were radiolabelled and analysed in the same way. Thus, once equilibrated to ambient temperature, each kit was reconstituted with 2 ml of sodium $^{99m}$Tc-pertechnetate solution containing 1.5 GBq (± 10 %) of radioactivity (1.5 GBq corresponds to 2 patient doses of 740 MBq), heated in a boiling water bath for 20 minutes and then cooled for 10 minutes before RCP analysis by HPLC and ITLC. Time of analysis reported as 'post-preparation'.

RCP determination
HPLC:

| | |
|---|---|
| Column: | Xterra $RP_{18}$ 3.5µm 3.0 x 50mm. Part number: 186000416 |
| Loop size: | 50µL |
| Mobile Phase: | 60% 50mM Ammonium Acetate pH 7: 40% Acetonitrile |
| Flow rate: | 0.5ml/min |

ITLC:
Pall ITLC-SG sheet (part number 61886) cut into strips 20mm x 200mm and eluted with 50% 1M Ammonium Acetate : 50% Acetone
RCP calculation:

$$RCP = (A+B)*((100-RHT)/100)$$

A = species A from HPLC, B = species B from HPLC, RHT = reduced hydrolysed technetium, species at origin from ITLC.
Species A and Species B are the diastereomers of $^{99m}$Tc-TRODAT as described by Meegalla *et al* [J.Med.Chem., 41, 428-436 (1998)].

**Example 3: Comparative kit performance for different generator elution conditions.**

Kits of formulations P and Q (as described in Example 1) were reconstituted, heated and analysed in exactly the same way, as per Example 2. Four generator elution conditions were investigated:

Generator elution conditions (1 to 4).

1. Fresh eluate: 24 hrs between elutions, <2hour old eluate;
2. Aged eluate: 24 hrs between elutions, >6 hour old eluate - high level of radiolysis products;
3. fresh eluate: 72 hrs between elutions, <2hour old eluate - low $^{99m}$Tc/$^{99}$Tc ratio);
4. aged eluate: 72 hrs between elutions, >6hour old eluate - low $^{99m}$Tc/$^{99}$Tc ratio and high level of radiolysis products.

RCP determinations were carried out at two post-preparation time points. The results are shown in Table 2:

Table 2:

| Radiolabelling of Formulations P and Q under four different generator elution conditions. | | | | |
|---|---|---|---|---|
| Generator Elution Condition | Formulation | Time Post Preparation (h & min) | Mean %RCP (S.D.) | Mean A:B ratio |
| **(1)** 24hr, <2hr | P | 0h 2 min | 91.7 (1.2) | 45:55 |
| | | 3h 27 min | 90.3 (1.1) | 47:53 |
| | Q | 0h 1 min | 88.3 (1.9) | 46:54 |
| | | 3h 28 min | 77.0 (1.5) | 56:44 |
| **(2)** 24hr, >6hr | P | 0h 4 min | 92.3 (1.2) | 45:55 |
| | | 4h 2 min | 90.1 (1.0) | 50:50 |
| | Q | 0h 1 min | 88.0 (1.6) | 47:53 |
| | | 3h 59min | 75.1 (0.8) | 58:42 |
| **(3)** 72hr, <2hr | P | 0h 1 min | 91.0 (0.6) | 45:55 |
| | | 4h 9 min | 88.3 (0.6) | 53:47 |
| | Q | 0h 2 min | 87.2 (2.3) | 46:54 |
| | | 2h 10 min | 73.0 (2.1) | 56:44 |
| **(4)** 72hr, >6hr | P | 1h 40 min | 89.8(1.1) | 46:54 |
| | | 3h 25 min | 88.7 (1.4) | 49:51 |
| | Q | 1h 40 min | 76.2 (3.2) | 56:44 |
| | | 3h 30 min | 70.5 (2.2) | 61:39 |

**Example 4: Effect of sodium *para*-aminobenzoate radioprotectant.**

A freshly prepared, nitrogen purged solution of sodium p-ABA (sodium para-aminobenzoate was added to a kit of Formulation Q. Radiolabelling of the kits was performed by first adding the radioprotectant (0.2 ml), followed by the immediate addition of pertechnetate solution (1GBq in 1.8 ml). The kits where then heated as described in Example 2. The results are given in Table 3:

Table 3:

| Effect of addition of p-ABA radioprotectant. | | | |
|---|---|---|---|
| **Batch** | **Radioprotectant** | **RCP at 1 hour** | **RCP at 4 hours** |
| TRD009 | p-ABA (200ug) | 90(n=2) | 86 (n=2) |
| TRD009 | None | 84 (n=2) | 76 (n=2) |
| | | | |
| TRD018 | p-ABA (200ug) | 88 (n=2) | 84 (n=2) |
| TRD018 | None | 85 (n=2) | 77 (n=2) |
| | | | |
| TRD018 | p-ABA (500ug) | 89 (n=2) | 87 (n=2) |
| TRD018 | None | 85 (n=2) | 78 (n=2) |

**Example 4: Effects of Reconstitution Volume.**

A comparison of the effects of reconstitution volume on the radiolabelling performance of the P and Q Formulations was carried out. Kits of both formulations were reconstituted, heated and analysed as per Examples 1 and 2. The radioactive concentration of eluate used to reconstitute the kits was kept constant at 1.5GBq/ml for each test item and eluate reconstitution volumes of 2, 4 and 6 ml were investigated.

Table 4:

| Effect of reconstitution volume on RCP of Formulations P and Q. | | | | |
|---|---|---|---|---|
| Activity/Volume | Formulation | Time post-preparation (h & mins) | Mean %RCP (S.D.) | Mean A:B |
| 1.5 GBq / 2 ml | P (n=2) | 0h 4min | 91.4 (0.8) | 45:55 |
| | | 4h 6min | 88.7 (1.2) | 46:54 |
| | Q (n=2) | 0h 4min | 84.9 (0.4) | 48:52 |
| | | 4h 6min | 70.0 (2.8) | 58:42 |
| 3 GBq / 4 ml | P (n=2) | 0h 6min | 92.6 (1.5) | 45:55 |
| | | 4h 7min | 88.7 (0.4) | 50:50 |
| | Q (n=2) | 0h 5min | 81.7 (3.1) | 47:53 |
| | | 4h 9min | 60.7 (0.1) | 60:40 |
| 4.5 GBq / 6 ml | P (n=2) | 0h 4min | 77.2 (5.5) | 54:46 |
| | | 4h 7min | 74.0 (3.2) | 68:32 |
| | Q (n=2) | 0h 4min | 70.6 (1.8) | 47:53 |
| | | 4h 5min | 41.6 (10.6) | 61:39 |

**Example 6: Study of the effect of heating using an autoclave.**

2 vials each of Formulations P and Q were reconstituted in the standard manner with 2 ml of sodium pertechnetate solution containing 1.5 GBq of radioactivity. The vials were subjected to an autoclave cycle of 121 °C for 25

minutes. The total duration of the cycle (heating and cooling) was about 120 minutes. As a result the earliest RCP analysis time point acquired was 2h 20 min post-reconstitution. Analysis times are reported in Table 5 as post-reconstitution (as opposed to post-preparation) time points:

Table 5:

| Comparative RCP of Formulations P and Q following an autoclave heating cycle (121 °C, 25 min). | | | |
|---|---|---|---|
| **Formulation** | **Time Post Reconstitution (hr &min)** | **Mean %RCP** | **Mean Ratio A:B** |
| P | 2h 35 min | 76.6 (n=2) | 50:50 |
| | 5h 43 min | 75.1 (n=2) | 55:45 |
| Q | 3h 03 min | 77.1 (n=2) | 54:46 |
| | 5h 40 min | 63.7 (n=2) | 60:40 |
| Control (P not autoclaved) | 0h 12 min | 89.1 (n=1) | 48:52 |

**Example 7: Study of the effect of an added radioprotectant on the biodistribution of $^{99m}$Tc-TRODAT.**

Kit formulation P was reconstituted to give $^{99m}$Tc-TRODAT as described in Examples 1 and 2, which was the Test Item. The radiochemical purity (RCP) of the Test Item was 92% pre-injection, falling to 91% by the post-injection analysis time point. At the pre- and post-injection analysis time points, there were a low percentage of lipophilic (approximately 2%) and hydrophilic (approximately 6%) radiolabelled species present. The ratio of the A and diastereomers (46:54) remained constant at the pre- and post-injection analysis time points. Experiments were performed at 6 predetermined time points (2 and 20 minutes, 1, 2, 4 and 7 hours) post injection (p.i.) of the Test Item in normal male Wistar rats (180 to 220 g). Animals were anaesthetised with Halothane (6 % in oxygen), injected with 0.1 ml (500 MBq/ml) Test Item, sacrificed, dissected and the samples assayed for radioactivity. A comparative study was carried out using a $^{99m}$Tc-TRODAT kit preparation corresponding to Formulation P, but lacking the ascorbate radioprotectant.

The percentage of the injected dose present in the blood was approximately three-fold lower for Formulation P at all the post-injection time points. The uptake and retention of radioactivity into the brain was similar at all except the 20 minute pi time point for both formulations. By 20 minutes pi, approximately 0.45% of the injected dose (id) was retained within the brain after administration of the radioprotectant formulation, relative to 0.29 % id after administration for the unstabilised formulation. This difference in brain uptake was reflected in the elevated percentage injected dose present in the brain regions at 20 minutes pi when expressed per gram of brain region.

The main difference observed was the elevated selective retention in the striatum after administration of the radioprotectant formulation, which peaked at $2.31 \pm 0.31$ after 2 hours pi and stayed at this peak level out to 4 hours pi ($2.42 \pm 0.80$). In comparison, after administration of the unstabilised formulation the selective retention in the striatum was $1.74 \pm 0.96$ by 2 hours pi and $0.76 \pm 0.30$ by 4 hours pi.

## Claims

**1.** A stabilised composition which comprises:

(i) a metal complex of a radioactive isotope of technetium or rhenium chelated to a conjugate, wherein said conjugate comprises a tetradentate chelating agent conjugated to a tropane, and said tetradentate chelating agent forms a neutral metal complex with said radioactive isotope of technetium or rhenium;
(ii) at least one radioprotectant.

**2.** The stabilised composition of claim 1, wherein the conjugate is of Formula Ia:

$$[\{tropane\}-(A)_n]_m-[tetradentate\ chelating\ agent] \quad (Ia)$$

where:

$-(A)_n-$ is a linker group wherein each A is independently $-CR_2-$, $-CR=CR-$, $-C{\equiv}C-$, $-CR_2CO_2-$, $-CO_2CR_2-$, $-NRCO-$, $-CONR-$, $-NR(C=O)NR-$, $-NR(C=S)NR-$, $-SO_2NR-$, $-NRSO_2-$, $-CR_2OCR_2-$, $-CR_2SCR_2-$,

$-CR_2NRCR_2-$, a $C_{4-8}$ cycloheteroalkylene group, a $C_{4-8}$ cycloalkylene group, a $C_{5-12}$ arylene group, or a $C_{3-12}$ heteroarylene group;
R is independently chosen from H, $C_{1-4}$ alkyl, $C_{2-4}$ alkenyl, $C_{2-4}$ alkynyl, $C_{1-4}$ alkoxyalkyl or $C_{1-4}$ hydroxyalkyl;
n is an integer of value 0 to 10; and,
m is 1, 2 or 3.

**3.** The stabilised composition of claims 1 or 2, where the tropane is a phenyl tropane of Formula III:

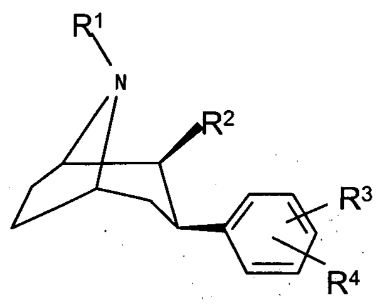

(III)

where:

$R^1$ is H, $C_{1-4}$ alkyl, $C_{1-4}$ alkenyl or $C_{1-4}$ fluoroalkyl;
$R^2$ is $CO_2R^5$, $CON(R^5)_2$, $COR^5$ or $C_{1-4}$ alkyl, where each $R^5$ is independently H or $C_{1-3}$ alkyl;
$R^3$ and $R^4$ are independently H, Cl, Br, F, I, $CH_3$, $CF_3$, $NO_2$, $OCH_3$ or $NH_2$.

**4.** The stabilised composition of claims 1 to 3, wherein the radioactive isotope of technetium or rhenium is $^{99m}Tc$.

**5.** The stabilised composition of claims 1 to 4, wherein the tetradentate chelating agent is conjugated to either the 2- or the 8- position of the tropane.

**6.** The stabilised composition of claim 5, wherein the tetradentate chelating agent is conjugated to the 2- position of the tropane.

**7.** The stabilised composition of claims 1 to 6, wherein the tetradentate chelating agent has a donor set chosen from:

(i) $N_2S_2$;
(ii) $N_3S$;
(iii) diaminedioxime, or
(iv) amidetriamine.

**8.** The stabilised composition of claims 1 to 7, wherein the tetradentate chelating agent has an $N_2S_2$ diaminedithiol donor set.

**9.** The stabilised composition of claims 1 to 8, wherein the radioprotectant comprises ascorbic acid, para-aminobenzoic acid or gentisic acid, or a salt thereof with a biocompatible cation.

**10.** The stabilised composition of claim 9, wherein the radioprotectant comprises ascorbic acid or a salt thereof with a biocompatible cation.

**11.** The stabilised composition of claim 10, wherein the radioprotectant comprises ascorbic acid or sodium ascorbate.

**12.** The stabilised composition of claims 1 to 11 wherein the metal complex comprises the radioactive technetium isotope $^{99m}Tc$;
the tetradentate chelating agent has an $N_2S_2$ diaminedithiol donor set and is conjugated to the 2-position of a phenyl tropane of Formula III;
and the radioprotectant comprises ascorbic acid or a salt thereof with a biocompatible cation.

**13.** The stabilised composition of claim 12, wherein the metal complex has the Formula:

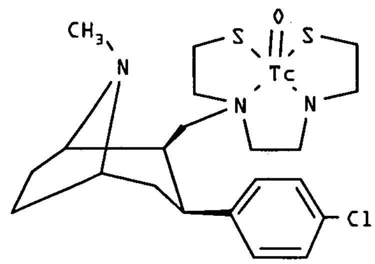

**14.** A conjugate useful in the preparation of the stabilised composition of claims 1 to 13, said conjugate being of Formula Ia as defined in claim 2.

**15.** The conjugate of claim 14, where the tropane of the conjugate of Formula Ia is a phenyl tropane of Formula III of claim 3.

**16.** The conjugate of claims 14 or 15 of Formula IV:

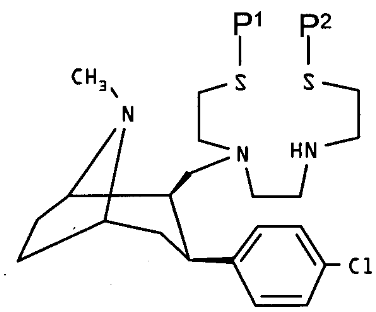

(IV)

where $P^1$ and $P^2$ are independently H or a thiol protecting group.

**17.** The conjugate of claim 16, where $P^1$ and $P^2$ are both H.

**18.** A radiopharmaceutical which comprises the stabilised composition of claims 1-13 together with a biocompatible carrier, in a form suitable for mammalian administration.

**19.** The radiopharmaceutical of claim 18, where the radiopharmaceutical is provided in a syringe.

**20.** The radiopharmaceutical of claim 18, where the radiopharmaceutical is provided in a vial fitted with a closure, wherein said closure is suitable for maintaining sterile integrity when punctured with a needle.

**21.** A kit for the preparation of the radiopharmaceutical of claims 18 to 20, which comprises:

(i) the conjugate of Formula (Ia) of claims 14 to 17 or a salt of said conjugate with a biocompatible counterion;
(ii) the radioprotectant of claim 1;
(iii) a biocompatible reductant.

**22.** The kit of claim 21, where the radioprotectant is as defined in claims 7 to 9.

**23.** The kit of claims 21 or 22, where the biocompatible reductant comprises stannous.

**24.** A method of preparation of the radiopharmaceutical of claims 18 - 20, which comprises forming the metal complex

of claims 1 to 8 in a biocompatible carrier in a form suitable for mammalian administration, and subsequently adding an effective amount of at least one radioprotectant.

**25.** The method of claim 24, where the metal complex comprises the conjugate of Formula Ia of claims 14 to 17.

**26.** The method of claims 24 or 25, where the radioprotectant is as defined in claims 7 to 9.

**27.** Use of the radiopharmaceutical of claims 18-20 in a method of diagnostic imaging of the mammalian body.

**28.** A method of diagnostic imaging of the mammalian body which comprises imaging a mammal which had previously been administered with the radiopharmaceutical of claims 18-20.

European Patent Office

# EUROPEAN SEARCH REPORT

Application Number
EP 03 00 2809

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 02 053192 A (AMERSHAM PLC)<br>11 July 2002 (2002-07-11)<br>* the whole document *<br>--- | 1-10 | A61K51/04<br>A61K49/00<br>A61K51/12<br>C07D451/02 |
| Y | WO 98 55154 A (COULTER PHARMACEUTICAL INC)<br>10 December 1998 (1998-12-10)<br>* claims 1-5,15 *<br>--- | 1-10 | |
| A | WO 01 40239 A (YALE UNIVERSITY)<br>7 June 2001 (2001-06-07)<br>* example 4 *<br>--- | 1-10 | |
| Y | EP 0 078 642 A (AMERSHAM INT PLC)<br>11 May 1983 (1983-05-11)<br>* the whole document *<br>--- | 1-9 | |
| Y | EP 0 004 684 A (PROCTER & GAMBLE)<br>17 October 1979 (1979-10-17)<br>* the whole document *<br>--- | 1-9 | |
| Y | GB 1 489 330 A (HOFFMANN LA ROCHE)<br>19 October 1977 (1977-10-19)<br>* the whole document *<br>--- | 1-10 | TECHNICAL FIELDS SEARCHED (Int.Cl.7)<br>A61K<br>C07D |
| D,Y | KUNG H F: "Development of Tc-99m labeled tropanes: TRODAT-1, as a dopamine transporter imaging agent"<br>NUCLEAR MEDICINE AND BIOLOGY, ELSEVIER SCIENCE PUBLISHERS, NEW YORK, NY, US,<br>vol. 28, no. 5, July 2001 (2001-07), pages 505-508, XP004247058<br>ISSN: 0969-8051<br>* figure 2 *<br>--- | 1-10 | |
| D,Y | US 5 980 860 A (KUNG HANK ET AL)<br>9 November 1999 (1999-11-09)<br>* figure 3 *<br>---<br>-/-- | 1-10 | |

~~The present search report has been drawn up for all claims~~

| Place of search | Date of completion of the search | Examiner |
|---|---|---|
| MUNICH | 7 October 2003 | Elliott, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 00 2809

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,Y | MELTZER PC ET AL: "A technetium-99m SPECT imaging agent which targets the dopamine transporter in primate brain" JOURNAL OF MEDICINAL CHEMISTRY, AMERICAN CHEMICAL SOCIETY. WASHINGTON, US, vol. 40, no. 12, 1997, pages 1835-1844, XP002194127 ISSN: 0022-2623 * page 1836 * --- | 1-10 | |
| D,Y | WO 97 16210 A (MASSACHUSETTS INSTITUTE OF TECHNOLOGY; ORGANIX INC; HARVARD) 9 May 1997 (1997-05-09) * the whole document * --- | 1-10 | |
| D,Y | MEEGALLA ET AL: "Synthesis and Characterization of Technetium-99m-Labeled Tropanes as Dopamine Transporter-Imaging Agents" JOURNAL OF MEDICINAL CHEMISTRY, AMERICAN CHEMICAL SOCIETY. WASHINGTON, US, vol. 40, no. 1, 1997, pages 9-17, XP002194129 ISSN: 0022-2623 * Scheme 3 * --- | 1-10 | TECHNICAL FIELDS SEARCHED (Int.Cl.7) |
| D,Y | FANG P ET AL: "Preparation and animal studies of [Tc-99m]-TRODAT-1 as a dopamine transporter imaging agent" CHINESE JOURNAL OF NUCLEAR MEDICINE, vol. 19, no. 3, August 1999 (1999-08), pages 146-8, XP009013496 * abstract * --- -/-- | 1-10 | |

~~The present search report has been drawn up for all claims~~

| Place of search | Date of completion of the search | Examiner |
|---|---|---|
| MUNICH | 7 October 2003 | Elliott, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 03 00 2809

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,Y | CHOI S R ET AL: "An improved kit formulation of a dopamine transporter imaging agent: [Tc-99m]TRODAT-1 - Transchelation preparation of Tc-99m-sestamibi" NUCLEAR MEDICINE AND BIOLOGY, ELSEVIER SCIENCE PUBLISHERS, NEW YORK, NY, US, vol. 26, no. 4, May 1999 (1999-05), pages 461-466, XP004167081 ISSN: 0969-8051 * figure 1 * --- | 1-10 | |
| D,Y | JURISSON S S ET AL: "Potential technetium small molecule radiopharmaceuticals" CHEMICAL REVIEWS, vol. 99, no. 9, September 1999 (1999-09), pages 2205-2218, XP000852431 ISSN: 0009-2665 * figure 2 * --- | 1-10 | |
| D,Y | TURPIN F ET AL: "Synthesis of two novel oxocyclam-binding technetium complexes containing an analogue of cocaine." JOURNAL OF LABELLED COMPOUNDS AND RADIOPHARMACEUTICALS, vol. 45, no. 5, April 2002 (2002-04), pages 379-393, XP002256764 April, 2002 ISSN: 0362-4803 * the whole document * --- | 1-10 | TECHNICAL FIELDS SEARCHED (Int.Cl.7) |
| D,Y | ARANO YASUSHI: "Delivery of diagnostic agents for gamma-imaging" ADVANCED DRUG DELIVERY REVIEWS, vol. 37, no. 1, 4 May 1999 (1999-05-04), pages 103-120, XP002256765 * figure 7 * --- -/-- | 1-10 | |

~~The present search report has been drawn up for all claims~~

| Place of search | Date of completion of the search | Examiner |
|---|---|---|
| MUNICH | 7 October 2003 | Elliott, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 00 2809

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| D,Y | MEEGALLA SANATH K ET AL: "Specificity of diastereomers of (99mTc)TRODAT-1 as dopamine transporter imaging agents." JOURNAL OF MEDICINAL CHEMISTRY, vol. 41, no. 4, 12 February 1998 (1998-02-12), pages 428-436, XP002256766 ISSN: 0022-2623 * Scheme 2 * ----- | 1-10 | |
| | | | TECHNICAL FIELDS SEARCHED (Int.Cl.7) |

~~The present search report has been drawn up for all claims~~

| Place of search | Date of completion of the search | Examiner |
|---|---|---|
| MUNICH | 7 October 2003 | Elliott, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number

EP 03 00 2809

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☒ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

## ANNEX TO THE EUROPEAN SEARCH REPORT ON EUROPEAN PATENT APPLICATION NO. EP 03 00 2809

This annex lists the patent family members relating to the patent documents cited in the above-mentioned European search report.
The members are as contained in the European Patent Office EDP file on
The European Patent Office is in no way liable for these particulars which are merely given for the purpose of information.

07-10-2003

| Patent document cited in search report | Publication date | Patent family member(s) | Publication date |
|---|---|---|---|
| WO 02053192 A | 11-07-2002 | CA 2431660 A1 | 11-07-2002 |
| | | EP 1345630 A1 | 24-09-2003 |
| | | WO 02053192 A1 | 11-07-2002 |
| | | NO 20032937 A | 25-08-2003 |
| WO 9855154 A | 10-12-1998 | US 5961955 A | 05-10-1999 |
| | | AU 743823 B2 | 07-02-2002 |
| | | AU 8057098 A | 21-12-1998 |
| | | EP 1003560 A1 | 31-05-2000 |
| | | JP 2002516612 T | 04-06-2002 |
| | | NO 995906 A | 03-12-1999 |
| | | NZ 502029 A | 21-12-2001 |
| | | WO 9855154 A1 | 10-12-1998 |
| | | US 6338835 B1 | 15-01-2002 |
| WO 0140239 A | 07-06-2001 | AU 4308001 A | 12-06-2001 |
| | | CA 2393610 A1 | 07-06-2001 |
| | | EP 1233968 A2 | 28-08-2002 |
| | | JP 2003515541 T | 07-05-2003 |
| | | WO 0140239 A2 | 07-06-2001 |
| | | US 2002111486 A1 | 15-08-2002 |
| EP 0078642 A | 11-05-1983 | AU 560024 B2 | 26-03-1987 |
| | | AU 8988882 A | 05-05-1983 |
| | | CA 1190473 A1 | 16-07-1985 |
| | | DE 3274621 D1 | 22-01-1987 |
| | | DE 78642 T1 | 27-10-1983 |
| | | EP 0078642 A2 | 11-05-1983 |
| | | FI 823657 A ,B, | 01-05-1983 |
| | | JP 1364440 C | 09-02-1987 |
| | | JP 58085823 A | 23-05-1983 |
| | | JP 61032291 B | 25-07-1986 |
| | | NO 823609 A ,B, | 02-05-1983 |
| | | US 4451451 A | 29-05-1984 |
| EP 0004684 A | 17-10-1979 | US 4233284 A | 11-11-1980 |
| | | AU 537689 B2 | 05-07-1984 |
| | | AU 4559579 A | 04-10-1979 |
| | | CA 1122524 A1 | 27-04-1982 |
| | | DE 2960805 D1 | 03-12-1981 |
| | | DK 133979 A ,B, | 01-10-1979 |
| | | EP 0004684 A2 | 17-10-1979 |
| | | IE 48095 B1 | 19-09-1984 |
| | | JP 1383535 C | 09-06-1987 |
| | | JP 55013258 A | 30-01-1980 |
| | | JP 61048485 B | 24-10-1986 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 03 00 2809

This annex lists the patent family members relating to the patent documents cited in the above-mentioned European search report.
The members are as contained in the European Patent Office EDP file on
The European Patent Office is in no way liable for these particulars which are merely given for the purpose of information.

07-10-2003

| Patent document cited in search report | | Publication date | Patent family member(s) | | | Publication date |
|---|---|---|---|---|---|---|
| EP 0004684 | A | | JP | 1049329 | B | 24-10-1989 |
| | | | JP | 1572270 | C | 25-07-1990 |
| | | | JP | 61210042 | A | 18-09-1986 |
| | | | NZ | 190089 | A | 19-11-1981 |
| | | | PH | 16295 | A | 05-09-1983 |
| | | | US | 4497744 | A | 05-02-1985 |
| | | | ZA | 7901470 | A | 28-05-1980 |
| GB 1489330 | A | 19-10-1977 | US | 4364920 | A | 21-12-1982 |
| | | | BE | 841261 | A1 | 29-10-1976 |
| | | | CA | 1114291 | A1 | 15-12-1981 |
| | | | CH | 626535 | A5 | 30-11-1981 |
| | | | DE | 2619382 | A1 | 11-11-1976 |
| | | | DK | 156976 | A ,B, | 31-10-1976 |
| | | | FR | 2309215 | A1 | 26-11-1976 |
| | | | JP | 1113829 | C | 16-09-1982 |
| | | | JP | 51148038 | A | 18-12-1976 |
| | | | JP | 57006409 | B | 04-02-1982 |
| | | | NL | 7604475 | A ,B, | 02-11-1976 |
| US 5980860 | A | 09-11-1999 | US | 6241963 | B1 | 05-06-2001 |
| | | | AU | 716235 | B2 | 24-02-2000 |
| | | | AU | 1156697 | A | 07-05-1997 |
| | | | CA | 2233173 | A1 | 24-04-1997 |
| | | | EP | 0929319 | A1 | 21-07-1999 |
| | | | JP | 11514368 | T | 07-12-1999 |
| | | | NZ | 324492 | A | 28-10-1999 |
| | | | WO | 9714445 | A1 | 24-04-1997 |
| WO 9716210 | A | 09-05-1997 | US | 6171576 | B1 | 09-01-2001 |
| | | | CA | 2236578 | A1 | 09-05-1997 |
| | | | EP | 0859641 | A1 | 26-08-1998 |
| | | | JP | 11515029 | T | 21-12-1999 |
| | | | US | 2002150535 | A1 | 17-10-2002 |
| | | | WO | 9716210 | A1 | 09-05-1997 |
| | | | US | 2003069269 | A1 | 10-04-2003 |
| | | | US | 6353105 | B1 | 05-03-2002 |
| | | | US | 6291512 | B1 | 18-09-2001 |
| | | | US | 6417221 | B1 | 09-07-2002 |

EPO FORM P0459

For more details about this annex : see Official Journal of the European Patent Office, No. 12/82